(19) Europäisches Patentamt
European Patent Office
Office européen des brevets

(11) Veröffentlichungsnummer: **0 330 940**
**A2**

(12) # EUROPÄISCHE PATENTANMELDUNG

(21) Anmeldenummer: **89102830.0**

(22) Anmeldetag: **18.02.89**

(51) Int. Cl.⁴: **C07D 295/14 , C07D 213/55 , C07C 101/18 , A61K 31/195 , A61K 31/40 , A61K 31/44**

(30) Priorität: **27.02.88 DE 3806321**

(43) Veröffentlichungstag der Anmeldung: **06.09.89 Patentblatt 89/36**

(84) Benannte Vertragsstaaten: **AT BE CH DE ES FR GB GR IT LI LU NL SE**

(71) Anmelder: **BOEHRINGER MANNHEIM GMBH**
**Sandhofer Strasse 116**
**D-6800 Mannheim 31(DE)**

(72) Erfinder: **Tsaklakidis, Christos, Dr.**
**Weberstrasse 23**
**D-6940 Weinheim(DE)**
Erfinder: **Leinert, Herbert, Dr.**
**Essigkamm 11**
**D-6148 Heppenheim(DE)**
Erfinder: **Freund, Peter, Dr.**
**Hebelstrasse 83**
**D-6834 Ketch(DE)**

(54) **Neue trisubstituierte Amine, Verfahren zu ihrer Herstellung sowie Arzneimittel, die diese Verbindungen enthalten.**

(57) Verbindungen der Formel I

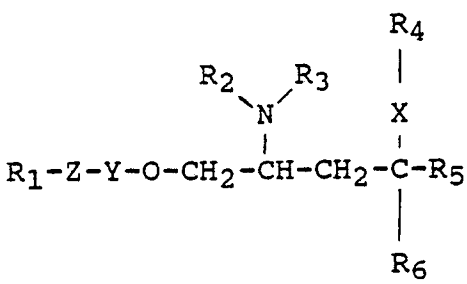

(I)

in der
$R_1$ Wasserstoff, einen Alkyl- oder Alkenylrest, einen Cycloalkyl- oder Cycloalkenylrest oder einen gegebenenfalls substituierten monocyclischen aromatischen oder heteroaromatischen Rest,
$R_2$ und $R_3$ einen Alkylrest oder zusammen einen Ring,
$R_4$ einen gegebenenfalls substituierten monocyclischen aromatischen oder heteroaromatischen Rest,
$R_5$ Wasserstoff, Nitril, Carbonsäureester oder einen gegebenenfalls substituierten monocyclischen aromatischen oder heteroaromatischen Rest,
$R_6$ Wasserstoff, Nitril, Carbonsäure, Carbonsäureester, Carbonsäureamid oder Hydroxymethyl,
X einen Valenzstrich oder die Methylengruppe,
Y einen Valenzstrich oder einen gegebenenfalls substituierten Alkyl- oder Alkenyl-Rest, und
Z einen Valenzstrich, ein Sauerstoffatom oder die Carbonylgruppe bedeuten,
sowie deren pharmakologisch verträgliche Salze und optische Isomere, Verfahren zu ihrer Herstellung und Arzneimittel, die diese Verbindungen enthalten, zur Behandlung von Herz- und Kreislauferkrankungen.

EP 0 330 940 A2

Xerox Copy Centre

## Neue trisubstituierte Amine, Verfahren zu ihrer Herstellung sowie Arzneimittel, die diese Verbindungen enthalten

Die vorliegende Erfindung betrifft neue trisubstituierte Amine, deren pharmakologisch verträgliche Salze, ihre Herstellung sowie diese enthaltende Arzneimittel.

Gegenstand der Erfindung sind neue Amino-Verbindungen der allgemeinen Formel I

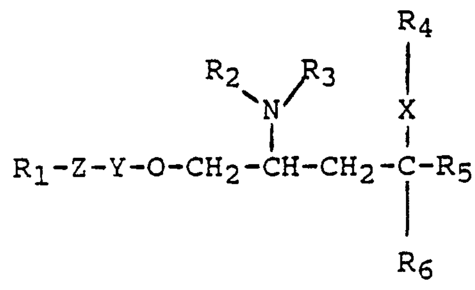 (I),

worin

$R_1$ Wasserstoff, einen geradkettigen oder verzweigten $C_1$-$C_{12}$-Alkylrest, einen $C_3$-$C_7$-Cycloalkylrest, einen geradkettigen oder verzweigten $C_2$-$C_{12}$-Alkenylrest oder einen unsubstituierten oder substituierten $C_3$-$C_7$-Mono- oder Bicycloalkenylrest bedeutet, einen unsubstituierten oder einen ein- oder mehrfach substituierten monocyclischen aromatischen oder heteroaromatischen Rest bedeutet,

$R_2$ und $R_3$ gleich oder verschieden sein können und einen geraden, verzweigten, gesättigten oder ungesättigten $C_1$-$C_6$ aliphatischen Rest bedeuten oder zusammen mit dem Stickstoffatom einen gesättigten oder ungesättigten Ring bilden, der noch weitere Heteroatome enthalten kann und gegebenenfalls durch eine niedrige Alkylgruppe, eine niedrige Alkoxygruppe oder ein Sauerstoffatom substituiert ist,

$R_4$ einen unsubstituierten oder einen ein- oder mehrfach substituierten monocyclischen aromatischen Rest, einen unsubstituierten oder substituierten fünf- oder sechsgliedrigen heteroaromatischen Rest bedeutet,

$R_5$ Wasserstoff, -C≡N,

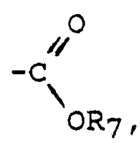

einen unsubstituierten, einen ein- oder mehrfach substituierten monocyclischen aromatischen Rest oder einen unsubstituierten oder substituierten fünf- oder sechsgliedrigen heteroaromatischen Rest bedeutet,

$R_6$ Wasserstoff, -C≡N,

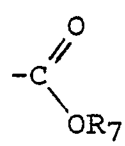 , 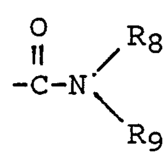 oder

$-CH_2-O-R_{10}$

$R_7$ Wasserstoff, einen $C_1$-$C_{12}$-Alkylrest oder einen N-Dialkylamino-$C_1$-$C_6$-alkyl-Rest bedeutet,

$R_8$ und $R_9$ gleich oder verschieden sein können und Wasserstoff einen geradkettigen, verzweigten, gesättigten oder ungesättigten aliphatischen $C_1$-$C_{12}$-Rest bedeuten, oder zusammen mit dem Stickstoff einen gesättigten oder ungesättigten Ring bilden mit 2 - 6 C-Atomen,

$R_{10}$ Wasserstoff, einen geradkettigen oder verzweigten $C_1$-$C_6$-Alkyl- oder $C_2$-$C_6$-Alkenylrest, einen Aralkylrest oder einen Acylrest bedeutet,

X einen Valenzstrich oder die Methylengruppe bedeutet,

Y einen Valenzstrich oder einen geradkettigen, verzweigten, gesättigten oder ungesättigten Kohlenwasserstoffrest von 1-6 Kohlenstoffatomen bedeutet, und

Z einen Valenzstrich, ein Sauerstoffatom oder die Carbonylgruppe bedeutet.
sowie deren pharmakologisch unbedenkliche Salze und optische Isomere.

Der $C_1$-$C_{12}$-Alkylrest von $R_1$ bedeutet vorzugsweise Methyl, Ethyl, Propyl, Isopropyl, Isobutyl, Isoamyl, Isohexyl, n-Hexyl, n-Octyl, n-Dodecyl, insbesondere Isobutyl, Isoamyl oder Isohexyl. Der $C_3$-$C_7$-Cycloalkylrest bedeutet in der Regel Cyclopentyl oder Cyclohexyl, $C_2$-$C_{12}$-Alkenylreste sind vorzugsweise Allyl, Methallyl, Isopentenyl oder Geranyl. Der $C_3$-$C_7$ Mono- oder Bicycloalkenylrest bedeutet in der Regel Cyclopentenyl, Cyclohexenyl oder Myrtenyl.
$R_2$ und $R_3$ bedeuten vorzugsweise Methyl, Ethyl, Propyl, Allyl oder Methallyl. Ringe, die $R_2$ und $R_3$ zusammen mit den Stickstoffatom, an das sie gebunden sind, bilden können, sind vorzugsweise der Pyrrolidin- oder der Piperidinring, insbesondere der Pyrrolidinring.
Die Heteroatome, die die Ringe enthalten können, sind Stickstoff, Schwefel oder Sauerstoff. Es sind hierunter Ringe wie z.B. Piperazin, Morpholin und Thiomorpholin zu verstehen. Substituenten der vorstehend genannten Ringe sind insbesondere $C_1$-$C_3$-Alkyl- oder $C_1$-$C_3$-Alkoxygruppen, wie z.B. Methyl, Ethyl oder Propyl oder Methoxy, Ethoxy oder Propoxy. Der Sauerstoff stellt in der Regel mit dem C-Atom, an das er gebunden ist, eine Carbonylgruppe dar. Entsprechende Ringe sind z.B. der Pyrrolidon- oder Piperidon-Ring.

Der unsubstituierte oder substituierte aromatische Rest der Reste $R_1$, $R_4$ und $R_5$ bedeutet Phenyl oder ein- oder mehrfach substituiertes Phenyl,
wobei als Substituenten insbesondere
$C_1$-$C_6$-Alkyl-, $C_2$-$C_6$-Alkenyl-, $C_1$-$C_6$-Alkoxy-, $C_2$-$C_6$-Alkenyloxy-, Hydroxyalkyl-, $C_1$-$C_6$-Alkylendioxy-, Hydroxy-alkoxy-, Alkoxy-alkoxy-, Alkylamino-, Dialkylamino-, Alkoxycarbonyl-alkoxy-, Phenylmercapto-, Alkylsulfinyl-, Alkylsulfonyl-, Alkylsulfonyloxy-carboxy-, Alkoxy-carbonyl-, Amino-carbonyl-, Mono- oder Dialkylamino-carbonyl-, Halogenalkyl- oder Cyano-gruppen sowie Halogenatome, wie Chlor, Brom oder Fluor in Frage kommen.

Heteroaromatische Reste der Substituenten $R_1$, $R_4$ und $R_5$ sind vorzugsweise Pyridyl, Pyrimidyl, Pyrazinyl, Thienyl, Oxazolyl, Pyrazolyl, Imidazolyl, Tetrazolyl, Thiazolyl, Isoxazolyl, insbesondere Pyridyl, Furanyl, Thienyl. Der $C_1$-$C_{12}$-Alkylrest von $R_7$, $R_8$ und $R_9$ bedeutet vorzugsweise Methyl, Ethyl, Propyl, Isopropyl, Isobutyl, Isoamyl, Isohexyl, n-Hexyl, n-Octyl, n-Dodecyl. Der N-Dialkylamino ($C_1$-$C_6$)-alkyl-Rest bedeutet in der Regel einen Methyl-, Ethyl-, Propyl- oder Hexylrest, der durch eine Dimethylamino oder Diethylamino-Gruppe substituiert ist, vorzugsweise einen Dimethylaminoethyl-Rest. Ringe, die $R_8$ und $R_9$ zusammen mit dem Stickstoffatom, an das sie gebunden sind, bilden können, sind vorzugsweise der Pyrrolidin- oder der Piperidinring.

Der $C_1$-$C_6$-Alkylrest des Substituenten $R_{10}$ bedeutet vorzugsweise Methyl, Propyl, Isobutyl. Der $C_2$-$C_6$-Alkylenrest bedeutet vorzugsweise Allyl, Methallyl oder Isobutenyl. Der Aralkylrest bedeutet in der Regel Benzyl- oder Picolyl. Acylreste sind bevorzugt Reste von aliphatischen $C_1$-$C_6$-Carbonsäuren, wie Formyl, Acetyl, Pivaloyl oder Arylcarbonsäuren, wie Benzoyl.

Bevorzugte Verbindungen der vorliegenden Erfindung sind Verbindungen der Formel I, in der
$R_1$ Isobutyl, Methallyl, Isopentenyl, Furyl, Thienyl, Pyridyl, Phenyl oder Phenyl, das durch Methyl, Methoxy oder Halogen substituiert ist,
$R_2$ und $R_3$ jeweils Ethyl oder zusammen mit dem Stickstoffatom, an das sie gebunden sind, einen Pyrrolidin-, Piperidin- oder Morpholin-Ring bilden,
$R_4$ Furyl, Thienyl, Pyridyl, Phenyl oder Phenyl, das durch Methyl, Methoxy oder Halogen substituiert ist,
$R_5$ Wasserstoff, Nitril, Ethoxycarbonyl, Furyl, Thienyl, Pyridyl, Phenyl oder Phenyl, das durch Methyl, Methylendioxy, Methoxy oder Halogen substituiert ist,
$R_6$ Wasserstoff, Nitril, Methoxycarbonyl, Ethoxycarbonyl, n-Propoxycarbonyl, Isopropoxycarbonyl, Aminocarbonyl, Diethylaminocarbonyl, Dimethylaminoethoxycarbonyl oder Piperidincarbonyl oder Hydroxymethyl,
X einen Valenzstrich oder die Methylengruppe,
Y einen Valenzstrich, eine Methylen- oder Ethylengruppe
und
Z einen Valenzstrich, ein Sauerstoffatom oder die Carbonylgruppe
bedeuten,
sowie deren pharmakologisch unbedenkliche Salze und optische Isomere. Die erfindungsgemäßen Verbindungen der allgemeinen Formel I können in an sich bekannter Weise dargestellt werden,

a) indem man eine Verbindung der allgemeinen Formel II,

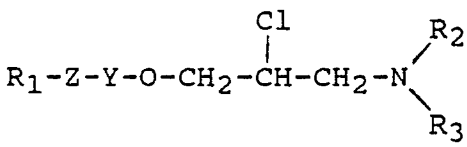

(II),

in der Y, Z, $R_1$, $R_2$ und $R_3$ die oben genannten Bedeutungen besitzen, mit einer Verbindung der allgemeinen Formel III,

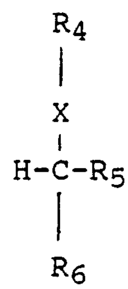

(III),

in der X, $R_4$, $R_5$ und $R_6$ die oben genannten Bedeutungen besitzen, umsetzt,
oder

b) eine Verbindung der allgemeinen Formel IV,

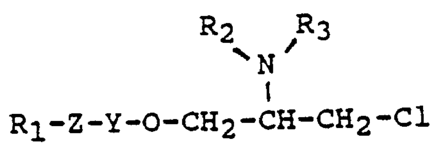

(IV),

in der Y, Z, $R_1$, $R^2$ und $R_3$ die obengenannten Bedeutungen besitzen, mit einer Verbindung der allgemeinen Formel III umsetzt
und anschließend gewünschtenfalls die erhaltenen Verbindungen in andere Verbindungen der Formel I überführt.

Die Umsetzung der Verbindungen der allgemeinen Formel II und IV mit einer Verbindung der allgmeinen Formel III zu den erfindungsgemäßen Verbindungen der allgmeinen Formel I erfolgt, in an sich bekannter Weise, in einem inerten Lösungsmittel wie Toluol, Xylol, Dimethylformamid oder Tetrahydrofuran bei Temperaturen zwischen 20 °C und Rückflußtemperatur des Lösungsmittels in Gegenwart eines alkalischen Kondensationsmittels, z.B. Natriumhydrid oder Lithiumdiisopropylamid.

Die Verbindungen der allgmeinen Formel II können dargestellt werden, indem man eine Verbindung der allgemeinen Formel V,

$R_1$-Z-Y-OH (V),

in der Y, Z, und $R_1$ die angegebene Bedeutung besitzen, mit Epichlorhydrin in Gegenwart von Natronlauge und eines Phasentransferkatalysators z.B. Tetrabutylammoniumbromid zu Reaktion bringt, die so erhaltenen Verbindungen der allgemeinen Formel VI

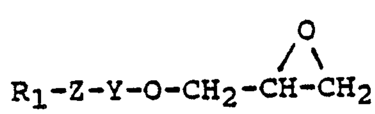

(VI),

mit einem Amin der allgemeinen Formel VII,

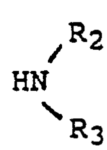

(VII),

in der $R_2$ und $R_3$ die oben angegebene Bedeutung besitzen, zum Alkohol der allgemeinen Formel VIII umsetzt und schließlich den Alkohol der allgemeinen Formel VIII

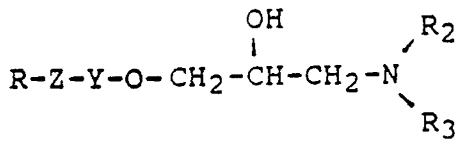

(VIII),

mit Thionylchlorid in einem inerten Lösungsmittel zur Reaktion bringt.

Die Verbindungen der allgemeinen Formel IV können dargestellt werden, daß man eine Verbindung der allgemeinen Formel IX

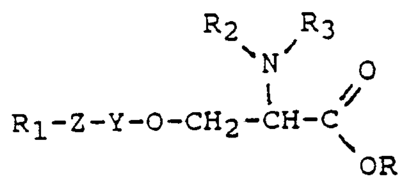

(IX),

in der Y, Z, $R_1$, $R_2$ und $R_3$ die oben angegebene Bedeutungen besitzen und R einen Alkylrest bedeutet, mit einem komplexen Hydrid, z.B. Lithiumaluminiumhydrid in einem inerten Lösungsmittel in an sich bekannter Weise zu einer Verbindung der allgemeinen Formel X

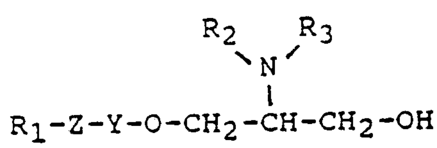

(X),

reduziert und diese in einem inerten Lösungsmittel mit Thionylchlorid chloriert.

Die nachträgliche Umwandlung von Verbindungen der Formel I in andere Verbindungen betrifft z. B. die Umwandlung des Substituenten $R_6$. Hierzu wird der üblicherweise eingesetzte Ester zur freien Carbonsäure verseift, oder zum entsprechenden Alkohol reduziert.

Die Ausgangsverbindungen der allgemeinen Formel IX können nach dem in der DE-B-2802864 beschriebenen Verfahren hergestellt werden.

Die erfindungsgemäßen Verbindungen der allgemeinen Formel I besitzen ein bis zwei asymmetrische Kohlenstoffatome.

Gegenstand der Erfindung sind daher auch Diastereomere, Racemate und die optisch aktiven Formen der erfindungsgemäßen Verbindungen der allgemeinen Formel I. Fallen Diastereomere bei der Synthese der erfindungsgemäßen Verbindungen an, so können diese durch Säulenchromatographie in den entsprechenden Racematen getrennt werden.

Die optisch aktiven Verbindungen können aus ihren racemischen Mischungen nach an sich bekannten Methoden über diastereomere Salze hergestellt werden. Zur Racematspaltung können z.B. Weinsäure, Apfelsäure, Camphersäure, Camphersulfonsäure oder Dibenzoylweinsäure verwendet werden.

Zur Überführung der Verbindungen der allgemeinen Formel I in ihre pharmakologisch unbedenklichen Salze setzt man diese, vorzugsweise in einem organischen Lösungsmittel, mit der äquivalenten Menge einer anorganischen oder organischen Säure, z.B. Salzsäure, Bromwasserstoffsäure, Phosphorsäure, Schwefelsäure, Essigsäure, Salicylsäure, Citronensäure, Benzoesäure, Naphthoesäure, o-Acetoxybenzoesäure, Adipinsäure, Maleinsäure oder Oxalsäure um.

Die erfindungsgemäßen Verbindungen der allgemeinen Formel I besitzen wertvolle pharmakologische Eigenschaften. Sie zeichnen sich besonders durch eine gefäßrelaxierende Wirkung aus und können daher zur Therapie von Herz-/Kreislauf-Erkrankungen eingesetzt werden.

Die erfindungsgemäßen neuen Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol,

Komplexbildner (wie Ethylendiamintetraessigsäure und deren nicht-toxischen Salze), hochmolekulare Polymere (wie flüssiges Polyethylenoxyd) zur Viskositätsregulierung. Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Fur die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Eine angemessene tägliche Dosis der aktiven Verbindung liegt zwischen 0,01 bis 50 mg/kg Köpergewicht, wobei diese in geeigneten Dosiseinheiten ein- oder mehrfach pro Tag verabreicht werden können.

Neben den nachfolgenden aufgeführten Beispielen sind insbesondere die folgenden Verbindungen im Sinne der Anmeldung bevorzugt:

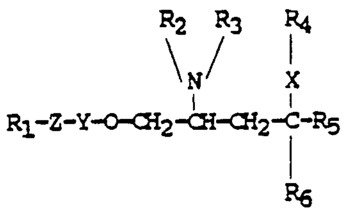

| Bsp. Nr. | $R_1$ | Z | Y | $R_2$, $R_3$ | $R_4$ | $R_5$ | $R_6$ | X |
|---|---|---|---|---|---|---|---|---|
| 1 | $(CH_3)_2-CHCH_2-$ | - | - | 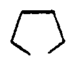 | 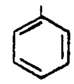 | 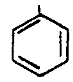 | -H | $-CH_2-$ |
| 2 | $(CH_3)_2-CHCH_2-$ | - | - | 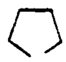 | 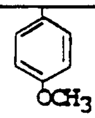 | 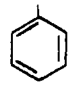 | 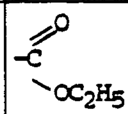 | $-CH_2-$ |
| 3 | $(CH_3)_2-CHCH_2-$ | - | - | 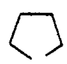 | 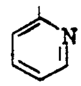 | 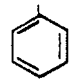 | 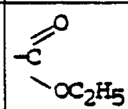 | $-CH_2-$ |
| 4 | $(CH_3)_2-CHCH_2-$ | - | - | 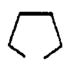 | 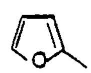 | 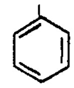 | 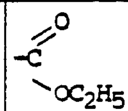 | $-CH_2-$ |
| 5 | $(CH_3)_2-CHCH_2-$ | - | - | 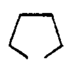 | 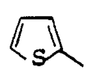 | 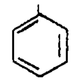 | 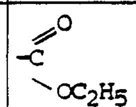 | $-CH_2-$ |
| 6 | $(CH_3)_2-CHCH_2-$ | - | - | 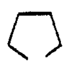 | 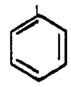 | 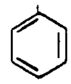 | 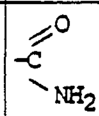 | - |
| 7 | $(CH_3)_2-CHCH_2-$ | - | - | $C_2H_5-$ | 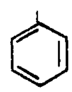 | 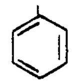 | -H | - |
| 8 | $(CH_3)_2-CHCH_2-$ | - | - | 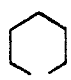 | 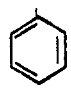 | 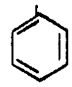 | -H | - |

Bsp.2: Öl, m/e 467

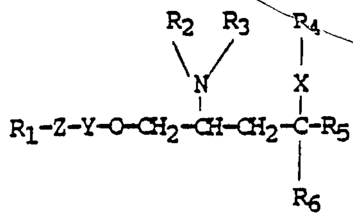

| Bsp. Nr. | $R_1$ | Z | Y | $R_2$, $R_3$ | $R_4$ | $R_5$ | $R_6$ | X |
|---|---|---|---|---|---|---|---|---|
| 9 | $(CH_3)_2-CHCH_2-$ | - | - | 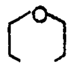 | 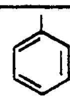 | 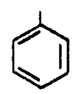 | -H | - |
| 10 | $(CH_3)_2-CHCH_2-$ | - | - | $C_2H_5-$ | 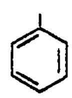 | 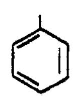 | 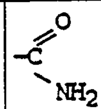 | - |
| 11 | $(CH_3)_2-CHCH_2-$ | - | - | 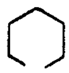 | 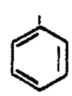 | 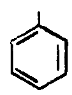 | 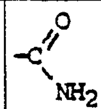 | - |
| 12 | $(CH_3)_2-CHCH_2-$ | - | - | 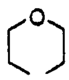 | 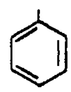 | 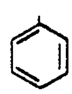 | 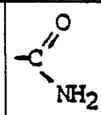 | - |
| 13 | $(CH_3)_2-CHCH_2-$ | - | - | $C_2H_5-$ | 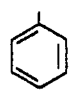 | 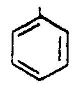 | $-C\equiv N$ | - |
| 14 | $(CH_3)_2-CHCH_2-$ | - | - | 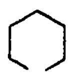 | 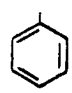 | 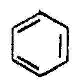 | $-C\equiv N$ | - |
| 15 | $(CH_3)_2-CHCH_2-$ | - | - | 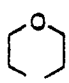 | 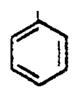 | 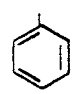 | $-C\equiv N$ | - |
| 16 | $(CH_3)_2-CHCH_2-$ | - | - | 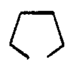 | 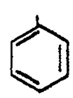 | 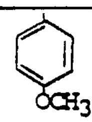 | -H | - |

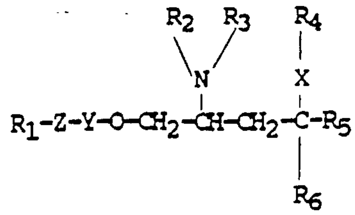

| Bsp. Nr. | $R_1$ | Z | Y | $R_2$, $R_3$ | $R_4$ | $R_5$ | $R_6$ | X |
|---|---|---|---|---|---|---|---|---|
| 17 | $(CH_3)_2-CHCH_2-$ | - | - | 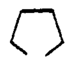 | 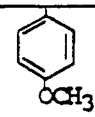 | 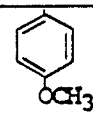 | -H | - |
| 18 | $(CH_3)_2-CHCH_2-$ | - | - | 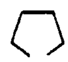 | 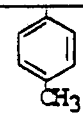 | 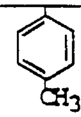 | -H | - |
| 19 | $(CH_3)_2-CHCH_2-$ | - | - | 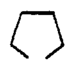 | 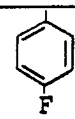 | 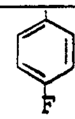 | -H | - |
| 20 | $(CH_3)_2-CHCH_2-$ | - | - | 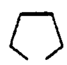 | 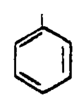 | 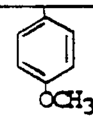 | 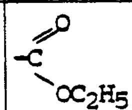 | - |
| 21 | $(CH_3)_2-CHCH_2-$ | - | - | 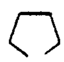 | 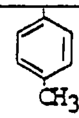 | 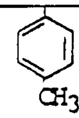 | 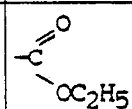 | - |
| 22 | $(CH_3)_2-CHCH_2-$ | - | - | 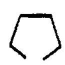 | 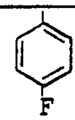 | 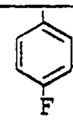 | 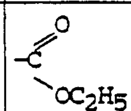 | - |
| 23 | $(CH_3)_2-CHCH_2-$ | - | - | 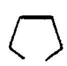 | 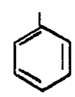 | 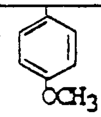 | 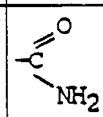 | - |
| 24 | $(CH_3)_2-CHCH_2-$ | - | - | 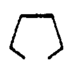 | 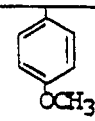 | 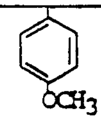 | 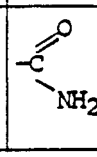 | - |

Bsp. 20: Öl, m/e 453

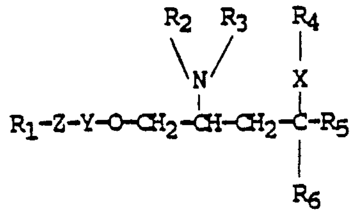

| Bsp. Nr. | $R_1$ | Z | Y | $R_2$, $R_3$ | $R_4$ | $R_5$ | $R_6$ | X |
|---|---|---|---|---|---|---|---|---|
| 25 | $(CH_3)_2-CHCH_2-$ | - | - | 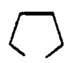 | 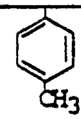 | 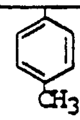 | 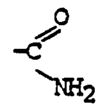 | - |
| 26 | $(CH_3)_2-CHCH_2-$ | - | - | 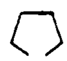 | 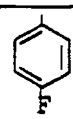 | 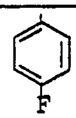 | 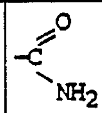 | - |
| 27 | $(CH_3)_2-CHCH_2-$ | - | - | 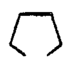 | 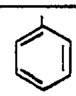 | 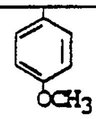 | $-C{\equiv}N$ | - |
| 28 | $(CH_3)_2-CHCH_2-$ | - | - | 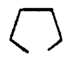 | 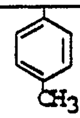 | 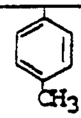 | $-C{\equiv}N$ | - |
| 29 | $(CH_3)_2-CHCH_2-$ | - | - | 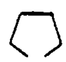 | 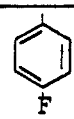 | 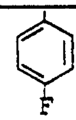 | $-C{\equiv}N$ | - |
| 30 | $(CH_3)_2-CHCH_2-$ | - | - | 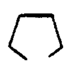 | 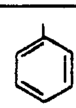 | 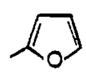 | -H | - |
| 31 | $(CH_3)_2-CHCH_2-$ | - | - | 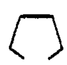 | 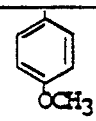 | 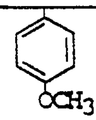 | 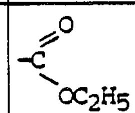 | $-CH_2-$ |
| 32 | 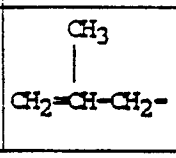 | - | - | 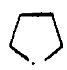 | 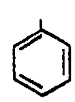 | 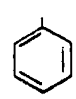 | -H | $-CH_2-$ |

Bsp. 27: Öl, m/e 406

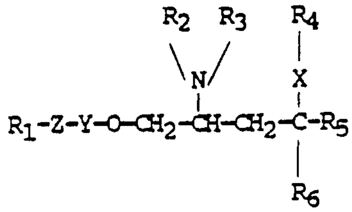

| Bsp. Nr. | $R_1$ | Z | Y | $R_2$, $R_3$ | $R_4$ | $R_5$ | $R_6$ | X |
|---|---|---|---|---|---|---|---|---|
| 33 | 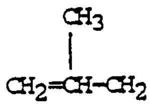 | - | - | 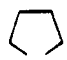 | 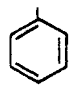 | 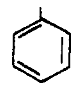 | -H | - |
| 34 | 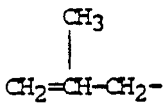 | - | - | 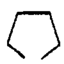 | 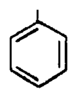 | 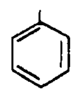 | 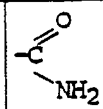 | - |
| 35 | 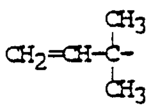 | - | - | 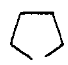 | 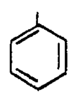 | 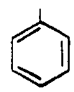 | -H | $-CH_2-$ |
| 36 | 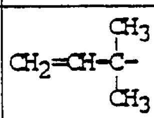 | - | - | 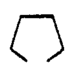 | 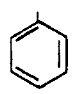 | 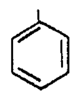 | 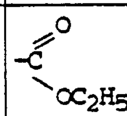 | $-CH_2-$ |
| 37 | 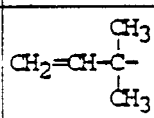 | - | - | 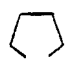 | 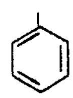 | 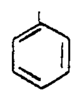 | -H | - |
| 38 | 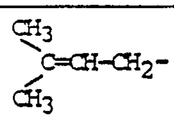 | - | - | 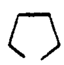 | 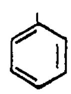 | 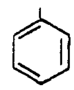 | -H | $-CH_2-$ |
| 39 | 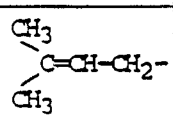 | - | - | 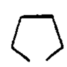 | 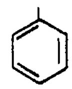 | 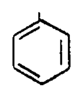 | -H | - |
| 40 | $(CH_3)_2-CHCH_2-$ | - | - | 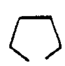 | 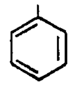 | 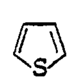 | -H | - |

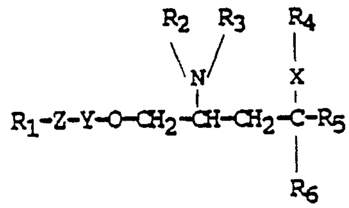

| Bsp. Nr. | $R_1$ | Z | Y | $R_2$, $R_3$ | $R_4$ | $R_5$ | $R_6$ | X |
|---|---|---|---|---|---|---|---|---|
| 41 | $(CH_3)_2-CHCH_2-$ | - | - | 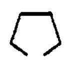 | 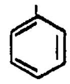 | 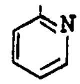 | -H | - |
| 42 | $(CH_3)_2-CHCH_2-$ | - | - | 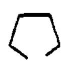 | 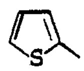 | 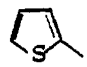 | -H | - |
| 43 | $(CH_3)_2-CHCH_2-$ | - | - | 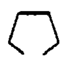 | 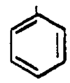 | 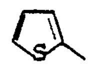 | 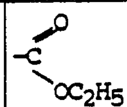 | - |
| 44 | $(CH_3)_2-CHCH_2-$ | - | - | 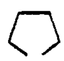 | 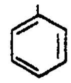 | 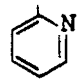 | 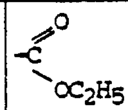 | - |
| 45 | $(CH_3)_2-CHCH_2-$ | - | - | 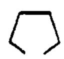 | 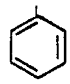 | 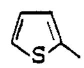 | 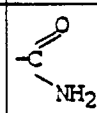 | - |
| 46 | $(CH_3)_2-CHCH_2-$ | - | - | 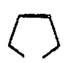 | 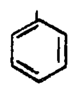 | 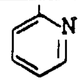 | 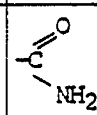 | - |
| 47 | $(CH_3)_2-CHCH_2-$ | - | - | 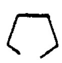 | 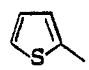 | 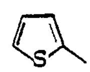 | 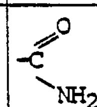 | - |
| 48 | $(CH_3)_2-CHCH_2-$ | - | - | 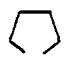 | 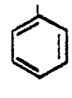 | 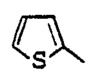 | $-C\equiv N$ | - |

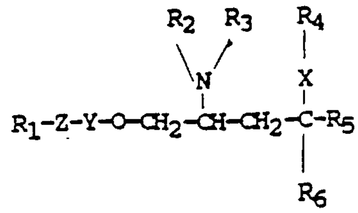

| Bsp. Nr. | $R_1$ | Z | Y | $R_2$, $R_3$ | $R_4$ | $R_5$ | $R_6$ | X |
|---|---|---|---|---|---|---|---|---|
| 49 | $(CH_3)_2-CHCH_2-$ | - | - | 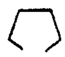 | 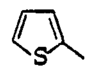 | 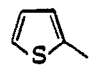 | $-C{\equiv}N$ | - |
| 50 | 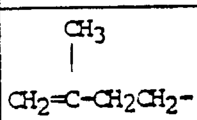 | - | - | 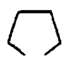 | 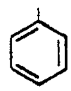 | 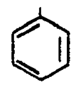 | -H | $-CH_2-$ |
| 51 | 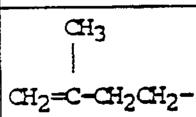 | - | - | 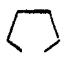 | 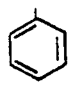 | 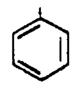 | -H | - |
| 52 | $(CH_3)_2-CHCH_2-$ | - | - | 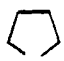 | 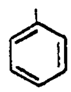 | 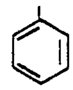 | 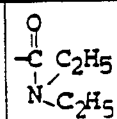 | - |
| 53 | $(CH_3)_2-CHCH_2-$ | - | - | 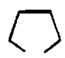 | 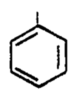 | 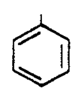 | 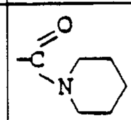 | - |
| 54 | -H | - | - | 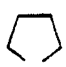 | 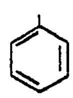 | 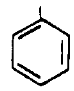 | 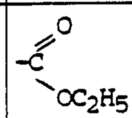 | $-CH_2-$ |
| 55 | 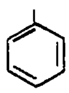 | 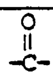 | - | 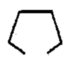 | 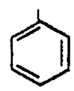 | 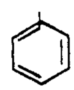 | 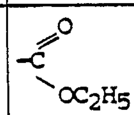 | $-CH_2-$ |
| 56 | 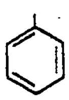 | - | $-CH_2-$ | 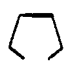 | 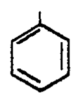 | 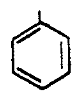 | 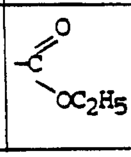 | $-CH_2-$ |

Bsp. 56: Öl, m/e 471

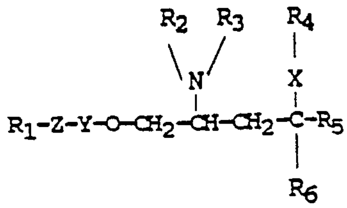

| Bsp. Nr. | R1 | Z | Y | R2, R3 | R4 | R5 | R6 | X |
|---|---|---|---|---|---|---|---|---|
| 57 | 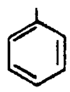 | - | - | 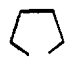 | 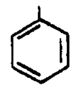 | 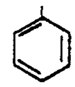 | -H | - |
| 58 | 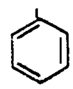 | - | $-CH_2-$ | 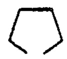 | 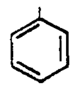 | 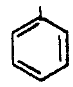 | -H | - |
| 59 | 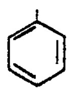 | - | - | 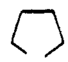 | 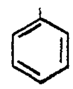 | 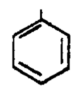 | 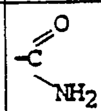 | - |
| 60 | 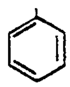 | - | $-CH_2-$ | 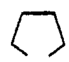 | 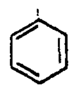 | 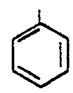 | 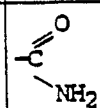 | - |
| 61 | 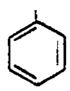 | - | $-CH_2-$ | 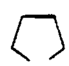 | 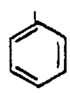 | 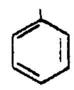 | $-C{\equiv}N$ | - |
| 62 | 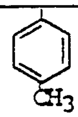 | - | $-CH_2-$ | 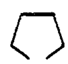 | 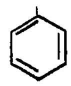 | 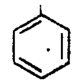 | 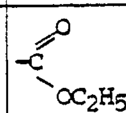 | - |
| 63 | 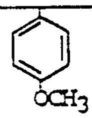 | - | $-CH_2-$ | 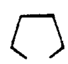 | 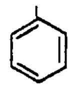 | 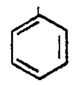 | 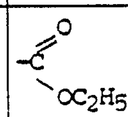 | - |
| 64 | 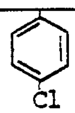 | - | $-CH_2-$ | 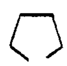 | 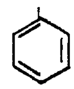 | 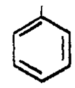 | 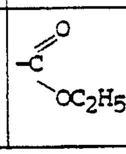 | - |

Bsp. 58: Öl, m/e 385
Bsp. 61: Öl, m/e 410
Bsp. 62: Öl, m/e 471
Bsp. 63: Öl, m/e 487
Bsp. 64: Öl, m/e 491

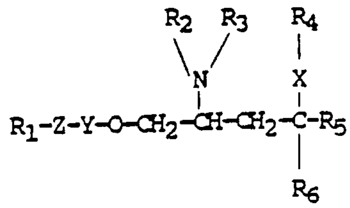

| Bsp. Nr. | $R_1$ | Z | Y | $R_2$, $R_3$ | $R_4$ | $R_5$ | $R_6$ | X |
|---|---|---|---|---|---|---|---|---|
| 65 | 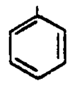 | - | $-(CH_2)_2-$ | 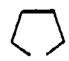 | 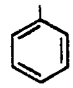 | 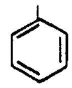 | 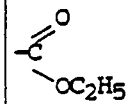 | - |
| 66 | 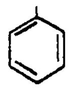 | - | $-CH_2-$ | 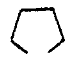 | 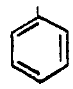 | 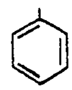 | 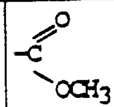 | - |
| 67 | 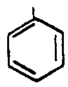 | - | $-CH_2-$ | 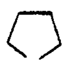 | 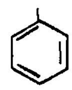 | 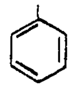 | 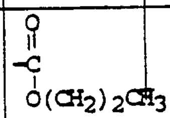 | - |
| 68 | 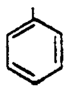 | - | $-CH_2-$ | 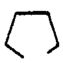 | 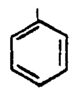 | 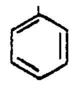 | 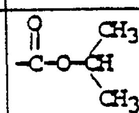 | - |
| 69 | 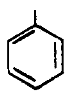 | - | $-CH_2-$ | 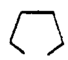 | 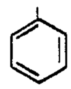 | 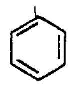 | 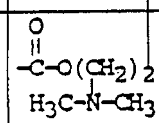 | - |
| 70 | 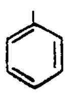 | O | $-(CH_2)_2$ | 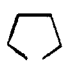 | 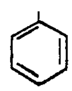 | 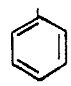 | 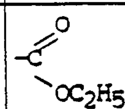 | - |
| 71 | 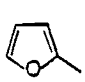 | - | $-CH_2-$ | 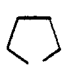 | 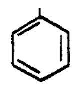 | 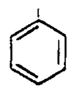 | 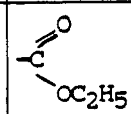 | - |
| 72 | 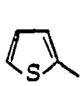 | - | $-CH_2-$ | 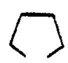 | 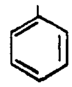 |  | 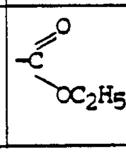 | - |

Bsp. 65: Öl, m/e 471
Bsp. 66: Öl, m/e 443
Bsp. 67: Öl, m/e 471
Bsp. 68: Öl, m/e 471
Bsp. 69: Oxalat, Fp.: 125° C, Essigester
Bsp. 70: Öl, m/e 487
Bsp. 71: Öl, m/e 447
Bsp. 72: Öl, m/e 449

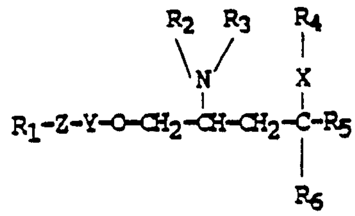

| Bsp. Nr. | $R_1$ | Z | Y | $R_2$, $R_3$ | $R_4$ | $R_5$ | $R_6$ | X |
|---|---|---|---|---|---|---|---|---|
| 73 | 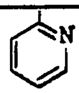 | - | $-CH_2-$ | 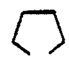 | 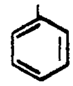 | 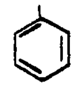 | -H | - |
| 74 | $(CH_3)_2-CHCH_2-$ | - | - | 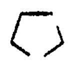 | 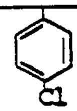 | 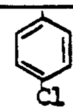 | -H | - |
| 75 | $(CH_3)_2-CHCH_2-$ | - | - | 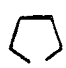 | 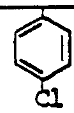 | 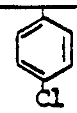 | 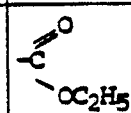 | - |
| 76 | $(CH_3)_2-CHCH_2-$ | - | - | 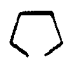 | 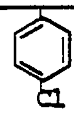 | 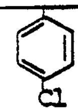 | 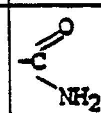 | - |
| 77 | $(CH_3)_2-CHCH_2-$ | - | - | 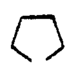 | 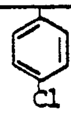 | 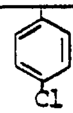 | $-C{\equiv}N$ | - |

Bsp. 73: Öl, m/e 386
Bsp. 74: Öl, m/e 420
Bsp. 75: Öl, m/e 492
Bsp. 77: Öl, m/e 445

Zur Charakterisierung der hergestellten Verbindungen wird in vielen Fällen der massenspektrometrisch bestimmte Molpeak m/e angegeben.

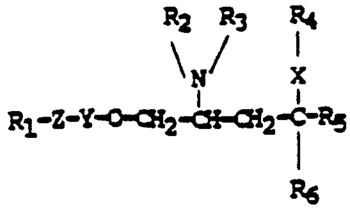

| Bsp. Nr. | $R_1$ | Z | Y | $R_2$, $R_3$ | $R_4$ | $R_5$ | $R_6$ | X |
|---|---|---|---|---|---|---|---|---|
| 78 | 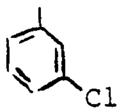 | - | $-CH_2-$ | 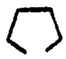 | 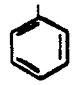 | 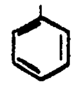 | $-C{\equiv}N$ | - |
| 79 | 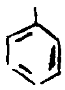 | - | $-CH_2-$ | 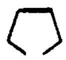 | 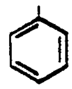 | 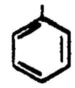 | $-CH_2-OH$ | - |
| 80 | 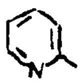 | - | $-CH_2-$ | 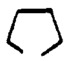 | 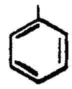 | 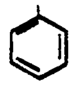 | $-CH_2-OCH_3$ | - |
| 81 | 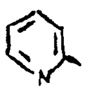 | - | $-CH_2-$ | 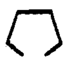 | 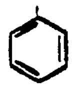 | 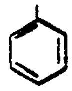 | $-CH_2-O-C_3H_7$ | - |
| 82 | 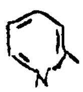 | - | $-CH_2-$ | 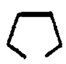 | 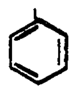 | 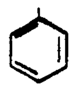 | 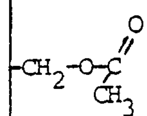 | - |
| 83 | 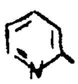 | - | $-CH_2-$ | 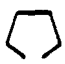 | 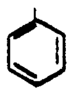 | 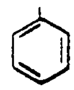 | 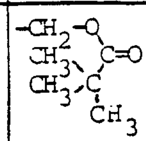 | - |
| 84 | 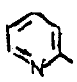 | - | $-CH_2-$ | 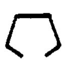 | 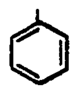 | 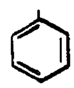 | 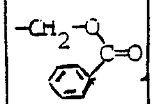 | - |
| 85 | 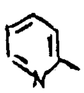 | - | $-CH_2-$ | 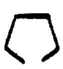 | 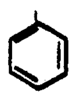 | 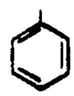 | 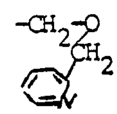 | - |

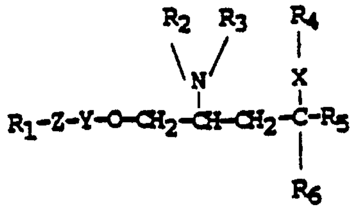

| Bsp. Nr. | $R_1$ | Z | Y | $R_2$, $R_3$ | $R_4$ | $R_5$ | $R_6$ | X |
|---|---|---|---|---|---|---|---|---|
| 86 | 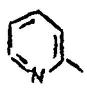 | - | $-CH_2-$ | 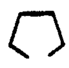 | 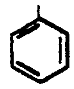 | 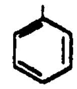 | -C≡N | - |
| 87 | 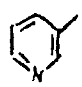 | - | $-CH_2-$ | 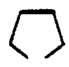 | 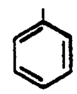 | 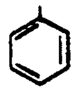 | -C≡N | - |
| 88 | 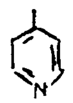 | - | $-CH_2-$ | 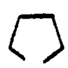 | 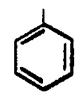 | 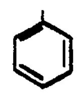 | -C≡N | - |
| 89 | 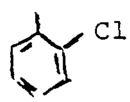 | - | $-CH_2-$ | 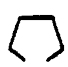 | 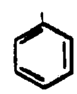 | 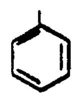 | 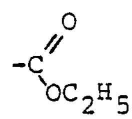 | - |
| 90 | 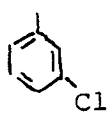 | - | $-CH_2-$ | 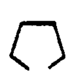 | 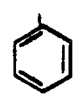 | 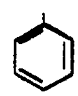 | 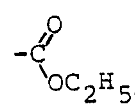 | - |
| 91 | 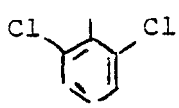 | - | $-CH_2-$ | 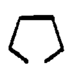 | 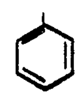 | 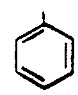 | 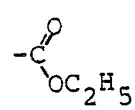 | - |
| 92 | 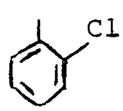 | - | $-CH_2-$ | 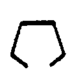 | 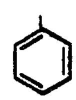 | 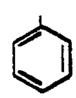 | -C≡N | - |
| 93 | 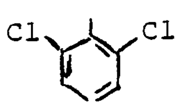 | - | $-CH_2-$ | 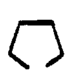 | 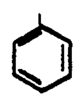 | 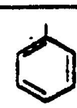 | -C≡N | - |

Beispiel 1

2,2-Bis-(4-Methoxyphenyl)-4-(1-pyrrolidino)-5-isobutoxyvaleriansäureethylester

Zu der eisgekühlten Suspension von 5.9 g (0.15 mol) Lithiumaluminiumhydrid in 250 ml abs. Tetrahydrofuran tropft man unter Rühren die Lösung von 27.1 g (0.1 mol) 2-(1-Pyrrolidino)-3-isobutoxy-propionsäureisobutylester (vgl. DE-B 2802864) in 100 ml abs. Tetrahydrofuran so zu, daß die Reaktionstemperatur nicht über 10 °C ansteigt. Nach beendete Zugabe läßt man das Reaktionsgemisch noch 30min bei Raumtemperatur rühren, versetzt es anschließend mit 40 ml Wasser, saugt ab, wascht den Niederschlag dreimal mit je 50 ml Tetrahydrofuran nach und zieht aus den vereinigten Filtraten das Tetrahydrofuran im Vacuum ab. Das zurückgebliebene gelbe Öl wird dann im Vacuum destilliert. Man erhält dabei als Hauptfraktion ($Kp_{0.05}$: 90 °C) 17.5 g (87 %) 1-Isobutoxy-2-(1-Pyrrolidino)-3-hydroxy-propan.

Zu der bei 0 °C gekühlten Lösung von 16.1 g (0.08 mol) 1-Isobutoxy-2-(1-Pyrrolidino)-3-hydroxy-propan in 100 ml 1,2-Dichlorethan tropft man 7.6 ml (12.4 g = 0.1 mol) Thionylchlorid in 50 ml 1,2-Dichlorethan zu. Nach beendeter Zugabe läßt man das Reaktionsgemisch noch 3 h bei Raumtemperatur rühren, zieht dann das überschüssige Thionylchlorid und das Lösungsmittel am Rotationsverdampfer ab und nimmt den Rückstand in 100 ml Methylenchlorid auf. Die Methylenchloridlösung wird anschließend zweimal mit je 50 ml gesätt. Natriumhydrogencarbonat-Lösung und einmal mit Wasser gewaschen, über Natriumsulfat getrocknet und im Vakuum eingeengt. Die Vakuumdestillation des Rohproduktes ($Kp_{0.06}$: 78 °C) ergibt 12.5 g (71.2 %) 1-Isobutoxy-2-(1-pyrrolidino)-3-chlorpropan.

Zu der Suspension von 0.27 g NaH (0.011 mol) Natriumhydrid in 50 ml trock. Toluol und 5 ml trock. Dimethylformamid tropft man unter Rühren die Lösung von 3.3 g (0.011 mol) 4,4′-Dimethoxy-diphenylessigsäureethylester (Lit. *) )/in 10 ml trock. Toluol zu und rührt danach noch 30 min bei Raumtemperatur.

Anschließend fügt man eine Lösung von 2 g (0.009 M) 1-Isobutoxy-2-(1-pyrrolidino)-3-chlorpropan in 10 ml trock. Toluol hinzu und erhitzt das Reaktionsgemisch 2 h bei 100 °C. Nach dem Abkühlen zieht man das Toluol im Vacuum ab, versetzt den Rückstand mit 10 ml gesätt. Ammoniumchloridlosung und schüttelt die wäßrige Mischung dreimal mit je 20 ml Methylenchlorid aus. Nach dem Trocknen der vereinigten organischen Phasen über Natriumsulfat wird das Methylenchlorid am Rotationsverdampfer abgezogen und der gelbe ölige Rückstand saulenchromatographisch gereinigt. Ausbeute 2.3 g (53 %) (farbloses Öl) 2,2-Bis-(4-Methoxyphenyl)-4-(1-Pyrrolidino)-5-isobutoxy-valeriansäureethylester.

## Beispiel 2

### 2-Phenyl-2-(2-pyridyl)-4-(1-pyrrolidino)-5-isobutoxyvaleronitril

Zu der Suspension von 0.15 g (6.2 mmol) 100proz. Natriumhydrid in 20 ml trock. Toluol gibt man unter Rühren 1.1 g (5.7 mmol) 2-Pyridyl-phenyl-acetonitril [Lit.: Klosa, Arch.Pharm. 286/58, 435 (1953] in 5 ml trock. Toluol zu und erhitzt die Mischung 10 min auf 100 °C. Anschließend tropft man die Lösung von 1.1 g (5 mmol) 1-Isobutoxy-2-(1-pyrrolidino)-3-chlorpropan in 5 ml trock. Toluol zu und erhitzt die Reaktionsmischung 2 h am Rückfluß. Nach dem Abkühlen wird das Toluol am Rotationsverdampfer abgezogen, der Rückstand mit 10 ml gesätt. Ammoniumchloridlösung versetzt, die wäßrige Mischung dreimal mit je 20 ml Methylenchlorid geschüttelt und die vereinigten organischen Phasen über Natriumsulfat getrocknet. Nach Abziehen des Lösungsmittels und säulenschromatographischer Reinigung des Rohproduktes erhält man 1.2 g (64 %) (farbloses Öl) 2-Phenyl2-(2-pyridyl)-4-(1-pyrrolidino)-5-isobutoxy-valeronitril.

## Beispiel 3

### 2,2-Diphenyl-4-(N,N-diethyl)amino-5-isobutoxy-valeriansäure-ethylester

Zu der Mischung von 64 ml Isobutanol, 155 ml konz. Natronlauge und 2 g Tetrabutylammoniumbromid tropft man unter starkem Rühren 217 ml Epichlorhydrin so zu, daß die Reaktionstemperatur nicht über 40 °C ansteigt. Nach beendetem Zutropfen läßt man das Reaktionsgemisch noch 2 h bei Raumtemperatur rühren, versetzt es dann mit 500 ml Eiswasser, trennt die organische Phase ab, schüttelt die wäßrige Phase noch zweimal mit je 50 ml Methylenchlorid aus und trocknet die vereinigten organischen Phasen über Natriumsulfat. Nach Abziehen des Methylenchlorids am Rotationsverdampfer wird der Rückstand im Vacuum destilliert. Man erhält bei 40 Torr und 66-70 °C 66 g (72 %) Isobutylglycidether.

Die Lösung von 5.2 g (0.04 mol) Isobutylglycidether und 5.2 ml (0.05 mol) Diethylamin in 60 ml abs. Ethanol wird unter Stickstoff 20 h unter Rückfluß gekocht. Anschließend werden das Ethanol und der

*) A. Bistrzycki, I. Paulus, R. Perrin Chem.Ber. 44, 2606)

Überschuß an Diethylamin am Rotationsverdampfer abgezogen und der Rückstand säulenchromatographisch gereinigt. Man erhält 6.9 g (85 %) 1-Isobutoxy-2-hydroxy-3-(N,N-diethyl)amino-propan als farbloses Öl.

Zu der Lösung von 3 g (14.6 mmol) 1-Isobutoxy-2-hydroxy-3-(N,N-diethyl)amino-propan in 30 ml 1,2-Dichlorethan tropft man bei Raumtemperatur 1.2 ml (16.4 mmol) Thionylchlorid in 10 ml 1,2-Dichlorethan zu. Anschließend erhitzt man die Reaktionsmischung 2 h am Rückfluß, kühlt ab, schüttelt zweimal mit je 50 ml gesätt. Natriumhydrogencarbonatlösung und trocknet die organische Phase über Natriumsulfat. Nach Abziehen des Lösungsmittels wird der Rückstand an Kieselgel chromatographiert. Man erhält 2 g (62.5 %) 1-Isobutoxy-2-chlor-3-(N,N-diethyl)amino-propan als gelbes Öl.

Zu der gerührten Suspension von 200 mg (8.3 mmol) Natriumhydrid in 30 ml trock. Toluol und 1 ml Dimethylformamid gibt man 2 g (8.3 mmol) Diphenylessigsäureethylester in 10 ml trock. Toluol bei Raumtemperatur hinzu und erhitzt anschließend die Mischung 20 min auf 80 °C. Danach tropft man bei 80 °C die Lösung von 1.6 g (7.2 mmol) 1-Isobutoxy2-chlor-3-(N,N-diethyl)amino-propan in 5 ml trock. Toluol zu und erhitzt noch 2 h am Rückfluß. Nach dem Abkuhlen wird die Reaktionsmischung mit 20 ml gesätt. Ammoniumchloridlösung versetzt, die organische Phase abgetrennt und über Natriumsulfat getrocknet. Nach Abziehen des Lösungsmittels wird das Rohprodukt säulenchromatographisch gereinigt Man erhält 1.4 g (46 %) 2,2-Diphenyl-4-(N,N-diethyl)amino-5-isobutoxy-valeriansäureethylester als farbloses Öl.

Beispiel 4

2.2-Diphenyl-4-(1-pyrrolidino)-5-benzyloxy-valeriansäureethylester

Zu der Mischung von 7.6 g (70.4 mmol) Benzylalkohol und 0.2 g Tetrabutylammoniumbromid in 16 ml konz. Natronlauge tropft man unter starkem Rühren bei 15-20 °C innerhalb 20 min 21.7 ml (277 mmol) Epichlorhydrin zu. Anschließend rührt man die Reaktionsmischung noch 3 h bei 40 °C. Nach dem Abkühlen wird dann die Reaktionsmischung mit 50 ml Eiswasser versetzt, die organische Phase abgetrennt und die wäßrige Phase zweimal mit je 20 ml Methylenchlorid ausgeschüttelt. Nach Trocknen der vereinigten organischen Phasen über Natriumsulfat und Abziehen des Lösungsmittels am Rotationsverdampfer erhält man 11.5 g rohen Benzylglycidether. 11.5 g roher Benzylglycidether werden in 300 ml abs. Ethanol gelöst und mit 25 ml Pyrrolidin versetzt. Anschließend erhitzt man die Reaktionslösung 3 h am Rückfluß, zieht dann das Lösungsmittel und den Überschuß an Pyrrolidin am Rotationsverdampfer ab, versetzt den Rückstand mit 100 ml Wasser, schüttelt die wäßrige Lösung dreimal mit je 50 ml Methylenchlorid aus und trocknet die vereinigten organischen Phasen über Natriumsulfat. Nach Abziehen des Methylenchlorids am Rotationsverdampfer wird der Rückstand im Vacuum destilliert. Man erhält bei 0.05 Torr und 114-118 °C 15.2 g (92 %) 1-Benzyloxy-2-hydroxy-3-(1-pyrrolidino)-propan als farblose Flüssigkeit. Zu der Lösung von 3.4 g (14.5 mmol) 1-Benzyloxy-2-hydroxy3-(1-pyrrolidino)-propan in 30 ml 1,2-Dichlorethan tropft man bei Raumtemperatur 1.2 ml (16.4 mmol) Thionylchlorid in 10 ml 1,2-Dichlorethan zu. Anschließend erhitzt man die Reaktionslösung 2 h am Rückfluß, kühlt dann ab, schüttelt zweimal mit je 50 ml gesätt. Natriumhydrogencarbonatlösung und trocknet die organische Phase über Natriumsulfat. Nach Abziehen des Lösungsmittels wird der Rückstand an Kieselgel chromatographiert. Man erhält 2.5 g (68 %) 1-Benzyloxy-2-chlor-3-(1-pyrrolidino)-propan.

Zu der gerührten Suspension von 200 mg (8.3 mmol) Natrium hydrid in 30 ml trock. Toluol und 1 ml Dimethylformamid gibt man 2 g (8.3 mmol) Diphenylessigsäureethylester in 10 ml trock. Toluol bei Raumtemperatur hinzu und erhitzt anschließend die Mischung 20 min auf 80 °C. Danach tropft man bei 80 °C die Lösung von 2 g (7.9 mmol) 1-Benzyloxy-2chlor-3-(1-pyrrolidino)-propan in 5 ml trock. Toluol zu und erhitzt noch 2 h am Rückfluß. Nach dem Abkühlen wird die Reaktionsmischung mit 20 ml gesätt. Ammoniumchloridlösung versetzt, die organische Phase abgetrennt und über Natriumsulfat getrocknet. Nach Abziehen des Lösungsmittels wird das Rohprodukt säulenchromatographisch gereinigt. Man erhält 1.7 g (47 %) 2,2-Diphenyl-4-(1-pyrrolidino)-5-benzyloxy-valeriansäureethylester als farbloses Öl.

Beispiel 5

2.2-Diphenyl-4-(1-pyrrolidino)-5-(2-picolyl)oxy-valeriansäureethylester

Zu der Mischung von 50 ml (0,52 Mol) Pyridin-2-carbinol und 8 g (0,025 Mol) Tetrabutylammoniumbromid in 200 ml konz. Natronlauge tropft man unter starkem Rühren bei 15-20 °C 190 ml (2,4 Mol) Epichlorhydrin zu. Anschließend läßt man die Reaktionsmischung über Nacht bei Raumtemperatur rühren, versetzt sie dann mit 500 ml Eiswasser, trennt die organische Phase ab, extrahiert die wässrige dreimal mit Ether und trocknet die vereinigten organischen Phasen über Natriumsulfat. Nach Abziehen des Lösungsmittels und des Überschusses an Epichlorhydrin am Rotationsverdampfer erhält man 57,8 g rohen 2-Picolyglycidether. 57,8 g roher 2-Picolylglycidether werden in 100 ml Ethanol gelöst und mit 58 ml Pyrrolidin versetzt. Anschließend erhitzt man die Reaktionsmischung 1 Stunde bei 50 °C, zieht dann das Lösungsmittel und den Überschuß an Pyrrolidin ab und destilliert den Rückstand im Vakuum. Man erhält 57 g (69 %) 1-(2-Picolyl)oxy-2-hydroxy-3-(1-pyrrolidino)-propan. $Kp._{0,01}$ = 133 °C.

Zu der Lösung von 48 g (0,2 Mol) 1-(2-Picolyl)oxy-2-hydroxy-3-(1-pyrrolidino)-propan in 600 ml 1,2-Dichlorethan tropft man bei Raumtemperatur 18 ml Thionylchlorid in 100 ml 1,2-Dichlorethan zu. Anschliessend erhitzt man die Reaktionsmischung 4 h am Rückfluß, läßt sie dann abkühlen und gießt sie auf 500 ml Eiswasser. Danach trennt man die organische Phase ab und extrahiert sie zweimal mit je 50 ml ln Salzsäure. Die vereinigten wässrigen Phasen werden mit ln Natronlauge schwach alkalisch gestellt (pH 8 - 9). Nun schüttelt man die alkalische Lösung dreimal mit je 100 ml Methylenchlorid, trocknet die vereinigten organischen Phasen über Natriumsulfat und zieht schließlich das Lösungsmittel am Rotationsverdampfer ab. Man erhält 50 g (96,6 %) rohes 1-(2-Picolyl)oxy-2-chlor-3-(1-pyrrolidino)-propan. Man löst 10 g (0,04 Mol) 1-(2-Picolyl)oxy-2-chlor-3-(1-pyrrolidino)-propan und 12 g (0,05 Mol) Diphenylessigsäureethylester in 100 ml trock. Dimethylformamid, versetzt die Lösung mit 1,2 g (0,05 Mol) Natriumhydrid und erhitzt anschließend die Reaktionsmischung 1 h bei 80 °C. Danach wird die abgekühlte Reaktionslösung mit 200 ml gesättigter Ammoniumchloridlösung versetzt und dreimal mit je 50 ml Methylenchlorid ausgeschüttelt. Nach dem Trocknen der vereinigten organischen Phasen über Natriumsulfat und Abziehen des Lösungsmittels wird der Rückstand an Kieselgel chromatographiert. Man erhält 9,2 g (50,3 %) der Titelverbindung als hellgelbes Öl, das beim Anreiben kristallisiert. Fp. 68 °C.

Die folgenden Verbindungen werden analog hergestellt:

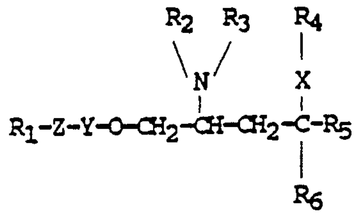

| Bsp. Nr. | $R_1$ | Z | Y | $R_2$, $R_3$ | $R_4$ | $R_5$ | $R_6$ | X |
|---|---|---|---|---|---|---|---|---|
| 1 | $(CH_3)_2-CHCH_2-$ | - | - | 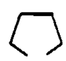 | 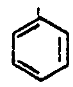 | 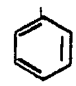 | 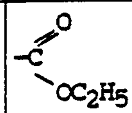 | $-CH_2-$ |
| 2 | $(CH_3)_2-CHCH_2-$ | - | - | 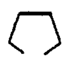 | 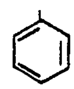 | -H | $-C{\equiv}N$ | - |
| 3 | $(CH_3)_2-CHCH_2-$ | - | - | 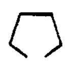 | 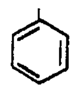 | -H | 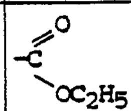 | - |
| 4 | $(CH_3)_2-CHCH_2-$ | - | - | 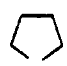 | 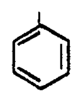 | 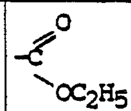 | 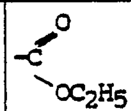 | - |
| 5 | $(CH_3)_2-CHCH_2-$ | - | - | 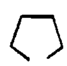 | 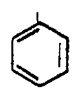 | $-C{\equiv}N$ | 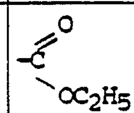 | - |
| 6 | $(CH_3)_2-CHCH_2-$ | - | - | 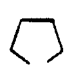 | 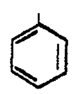 | 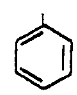 | -H | - |
| 7 | $(CH_3)_2-CHCH_2-$ | - | - | 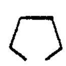 | 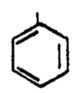 | 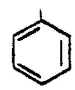 | 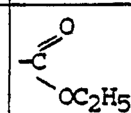 | - |
| 8 | $(CH_3)_2-CHCH_2-$ | - | - | 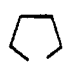 | 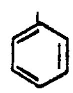 | 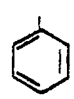 | $-C{\equiv}N$ | - |

Bsp. 1: Öl, m/e 437
Bsp. 2: Öl, m/e 300
Bsp. 3: Öl, m/e 347
Bsp. 4: Öl, m/e 419
Bsp. 5: Öl, m/e 372
Bsp. 6: Oxalat, 105° C, Essigester
Bsp. 7: Öl, m/e 423
Bsp. 8: Fest, 47° C, Heptan

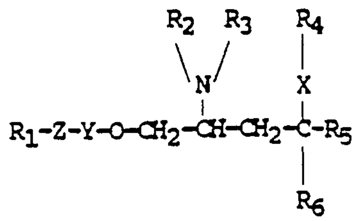

| Bsp. Nr. | $R_1$ | Z | Y | $R_2$, $R_3$ | $R_4$ | $R_5$ | $R_6$ | X |
|---|---|---|---|---|---|---|---|---|
| 9 | $(CH_3)_2$-$CHCH_2$- | - | - | 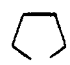 | 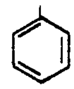 | 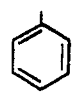 | 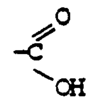 | - |
| 10 | $(CH_3)_2$-$CHCH_2$- | - | - | 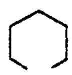 | 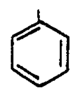 | 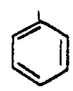 | 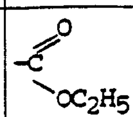 | - |
| 11 | $(CH_3)_2$-$CHCH_2$- | - | - | 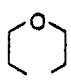 | 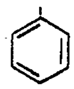 | 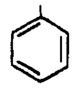 | 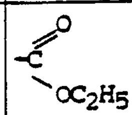 | - |
| 12 | $(CH_3)_2$-$CHCH_2$- | - | - | 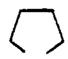 | 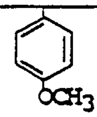 | 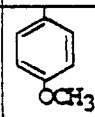 | -C≡N | - |
| 13 | $(CH_3)_2$-$CHCH_2$- | - | - | 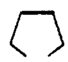 | 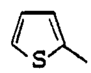 | 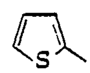 | 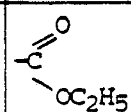 | - |
| 14 | 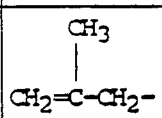 | - | - | 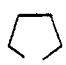 | 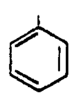 | 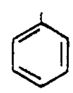 | 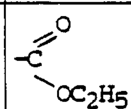 | - |
| 15 | 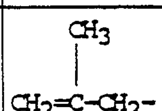 | - | - | 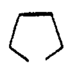 | 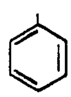 | 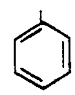 | -C≡N | - |
| 16 | 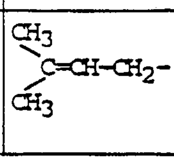 | - | - | 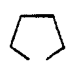 | 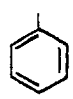 | 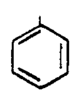 | 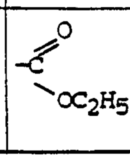 | $-CH_2-$ |

Bsp. 9: Fest, 198° C, Essigester
Bsp. 10: Öl, m/e 437
Bsp. 11: Öl, m/e 439
Bsp. 12: Öl, m/e 436
Bsp. 13: Öl, m/e 435
Bsp. 14: Öl, m/e 421
Bsp. 15: Öl, m/e 374
Bsp. 16: Öl, m/e 449

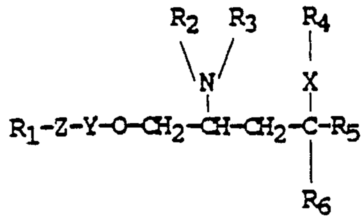

| Bsp. Nr. | $R_1$ | Z | Y | $R_2$, $R_3$ | $R_4$ | $R_5$ | $R_6$ | X |
|---|---|---|---|---|---|---|---|---|
| 17 | 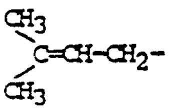 | - | - | 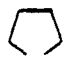 | 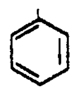 | 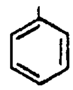 | 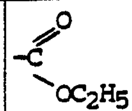 | - |
| 18 | 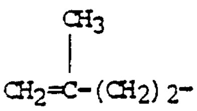 | - | - | 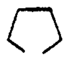 | 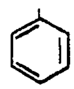 | 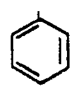 | 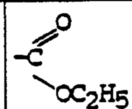 | $-CH_2-$ |
| 19 | 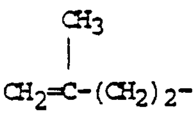 | - | - | 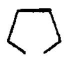 | 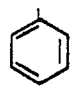 | 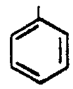 | 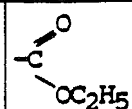 | - |
| 20 | 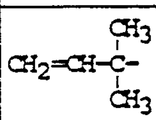 | - | - | 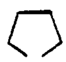 | 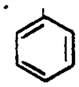 | 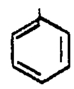 | 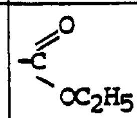 | - |
| 21 | 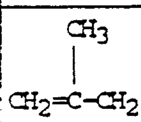 | - | - | 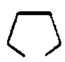 | 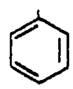 | 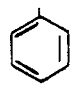 | 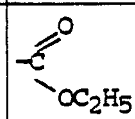 | $-CH_2-$ |
| 22 | 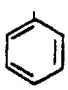 | - | - | 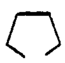 | 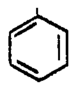 | 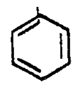 | 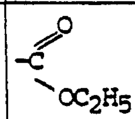 | - |
| 23 | 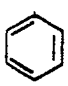 | - | - | 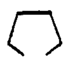 | 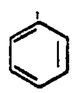 | 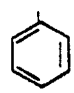 | $-C\equiv N$ | - |

Bsp. 17: Öl, m/e 435
Bsp. 18: Öl, m/e 449
Bsp. 19: Öl, m/e 435
Bsp. 20: Öl, m/e 435
Bsp. 21: Öl, m/e 435
Bsp. 22: Öl, m/e 443
Bsp. 23: Öl, m/e 396

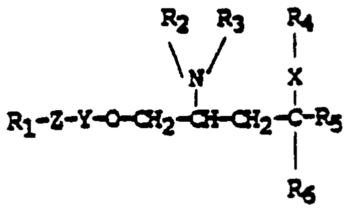

| Bsp. Nr. | R1 | Z | Y | R2, R3 | R4 | R5 | R6 | X |
|---|---|---|---|---|---|---|---|---|
| 24 | 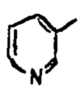 | - | $-CH_2-$ | 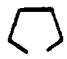 | 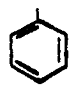 | 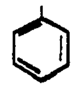 | 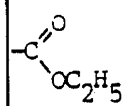 | - |
| 25 | 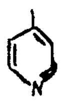 | - | $-CH_2-$ | 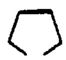 | 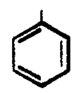 | 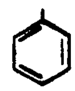 | 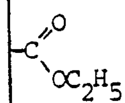 | - |
| 26 | 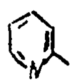 | - | $-CH_2-$ | 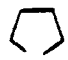 | 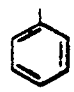 | 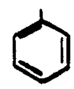 | 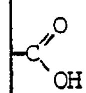 | - |
| 27 | 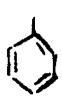 | - | $-CH_2-$ | 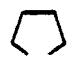 | 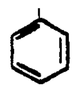 | 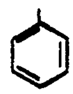 | 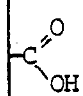 | - |
| 28 | 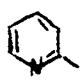 | - | - | 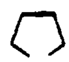 | 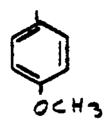 | 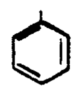 | 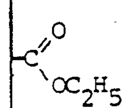 | $-CH_2-$ |
| 29 | $CH_3$<br>$CH-CH_2$<br>$CH_3$ | - | - | 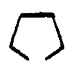 | 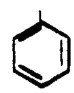 | 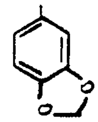 | 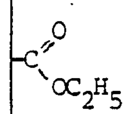 | $-CH_2-$ |
| 30 | 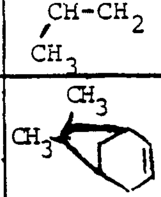 | - | $-CH_2-$ | 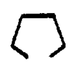 | 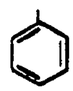 | 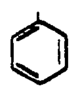 | 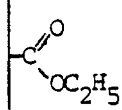 | - |
| 31 | 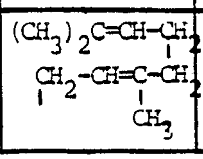 | - | - | 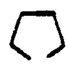 | 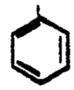 | 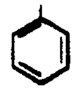 | 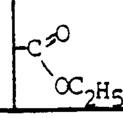 | - |

Bsp. 24: Öl, m/e 458
Bsp. 25: Öl, m/e 458
Bsp. 26: Fp: 180°C
Bsp. 27: Fp: 182°C
Bsp. 28: Öl, m/e 502
Bsp. 29: Öl, m/e 481
Bsp. 30: Öl, m/e 581
Bsp. 31: Öl, m/e 503

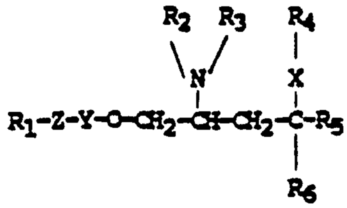

| Bsp. Nr. | $R_1$ | Z | Y | $R_2$, $R_3$ | $R_4$ | $R_5$ | $R_6$ | X |
|---|---|---|---|---|---|---|---|---|
| 32 | 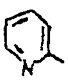 | - | $-CH_2-$ | 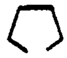 | 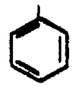 | 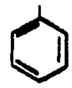 | $-CH_2OH$ | - |
| 33 | 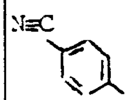 | - | $-CH_2-$ | 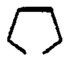 | 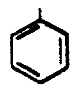 | 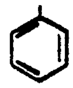 | 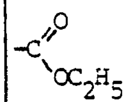 | - |
| 34 | 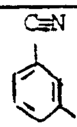 | - | $-CH_2-$ | 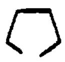 | 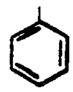 | 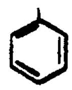 | 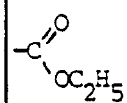 | - |
| 35 | 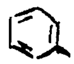 | - | $-CH_2-$ | 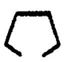 | 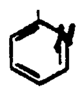 | 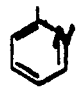 | $-C\equiv N$ | - |
| 36 | 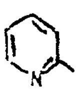 | - | $-CH_2-$ | 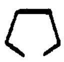 | 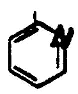 | 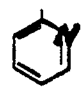 | $-C\equiv N$ | - |

Bsp. 32: Fp.: 63 - 65 °C
Bsp. 33: Öl, m/e 482
Bsp. 34: Öl, m/e 482
Bsp. 35: Öl, m/e 412
Bsp. 36: Öl, m/e 413

## Ansprüche

1. Verbindungen der Formel I

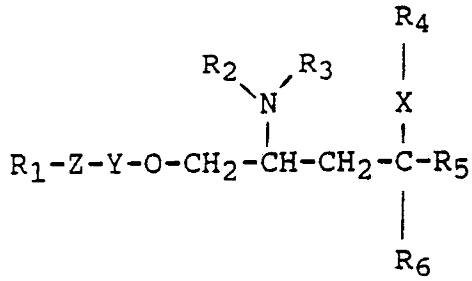

(I),

worin
$R_1$ Wasserstoff, einen geradkettigen oder verzweigten $C_1$-$C_{12}$-Alkylrest, einen $C_3$-$C_7$-Cycloalkylrest, einen geradkettigen oder verzweigten $C_2$-$C_{12}$-Alkenylrest oder einen unsubstituierten oder substituierten $C_3$-$C_7$ Mono- oder Bicycloalkenylrest bedeutet, einen unsubstituierten oder einen ein- oder mehrfach substituierten monocyclischen aromatischen oder heteroaromatischen Rest bedeutet,
$R_2$ und $R_3$ gleich oder verschieden sein können und einen geraden, verzweigten, gesättigten oder ungesättigten $C_1$-$C_6$ aliphatischen Rest bedeuten oder zusammen mit dem Stickstoffatom einen gesättigten oder ungesättigten Ring bilden, der noch weitere Heteroatome enthalten kann und gegebenenfalls durch eine niedrige Alkylgruppe, eine niedrige Alkoxygruppe oder ein Sauerstoffatom substituiert ist,
$R_4$ einen unsubstituierten oder einen ein- oder mehrfach substituierten monocyclischen aromatischen Rest, einen unsubstituierten oder substituierten fünf-oder sechsgliedrigen heteroaromatischen Rest bedeutet,
$R_5$ Wasserstoff, -C≡N,

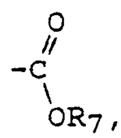

einen unsubstituierten, einen ein- oder mehrfach substituierten monocyclischen aromatischen Rest oder einen unsubstituierten oder substituierten fünf-oder sechsgliedrigen heteroaromatischen Rest bedeutet,
$R_6$ Wasserstoff, -C≡N,

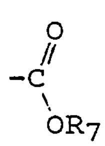 , 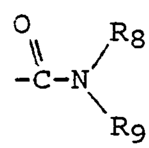 oder

$-CH_2-O-R_{10}$
$R_7$ Wasserstoff, einen $C_1$-$C_{12}$-Alkylrest oder einen N-Dialkylamino-$C_1$-$C_6$-alkyl-Rest bedeutet,
$R_8$ und $R_9$ gleich oder verschieden sein können und Wasserstoff einen geradkettigen, verzweigten, gesättigten oder ungesättigten aliphatischen $C_1$-$C_{12}$-Rest bedeuten, oder zusammen mit dem Stickstoff einen gesättigten oder ungesättigten Ring bilden mit 2 - 6 C-Atomen.
$R_{10}$ Wasserstoff, einen geradkettigen oder verzweigten $C_1$-$C_6$-Alkyl- oder $C_2$-$C_6$-Alkenylrest, einen Aralkylrest oder einen Acylrest bedeutet,
X einen Valenzstrich oder die Methylengruppe bedeutet,
Y einen Valenzstrich oder einen geradkettigen, verzweigten, gesättigten oder ungesättigten Kohlenwasserstoffrest von 1-6 Kohlenstoffatomen bedeutet, und
Z einen Valenzstrich, ein Sauerstoffatom oder die Carbonylgruppe bedeutet.
sowie deren pharmakologisch unbedenkliche Salze und optische Isomere.

2 .Verbindungen der Formel I gemäß Anspruch 1, in der

$R_1$ Isobutyl, Methallyl, Isopentenyl, Furyl, Thienyl, Pyridyl, Phenyl oder Phenyl, das durch Methyl, Methoxy oder Halogen substituiert ist,
$R_2$ und $R_3$ jeweils Ethyl oder zusammen mit dem Stickstoffatom, an das sie gebunden sind, einen Pyrrolidin-, Piperidin- oder Morpholin-Ring bilden,
$R_4$ Furyl, Thienyl, Pyridyl, Phenyl oder Phenyl, das durch Methyl, Methoxy oder Halogen substituiert ist,
$R_5$ Wasserstoff, Nitril, Ethoxycarbonyl, Furyl, Thienyl, Pyridyl, Phenyl oder Phenyl, das durch Methyl, Methylendioxy, Methoxy oder Halogen substituiert ist,
$R_6$ Wasserstoff, Nitril, Methoxycarbonyl, Ethoxycarbonyl, n-Propoxycarbonyl, Isopropoxycarbonyl, Aminocarbonyl, Diethylaminocarbonyl,
Dimethylaminoethoxycarbonyl, Piperidincarbonyl oder Hydroxymethyl,
X einen Valenzstrich oder die Methylengruppe,
Y einen Valenzstrich, eine Methylen- oder Ethylengruppe
und
Z einen Valenzstrich, ein Sauerstoffatom oder die Carbonylgruppe
bedeuten,
sowie deren pharmakologisch unbedenkliche Salze und optische Isomere.

3. Verfahren zur Herstellung von Verbindungen der Formel I

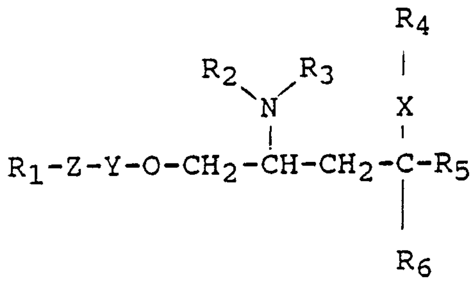

(I),

worin
$R_1$ Wasserstoff, einen geradkettigen oder verzweigten $C_1$-$C_{12}$-Alkylrest, einen $C_3$-$C_7$-Cycloalkylrest, einen geradkettigen oder verzweigten $C_2$-$C_6$-Alkenylrest oder einen $C_3$-$C_7$-Cycloalkenylrest bedeutet, einen unsubstituierten oder einen ein- oder mehrfach substituierten monocyclischen aromatischen oder heteroaromatischen Rest bedeutet,
$R_2$ und $R_3$ gleich oder verschieden sein können und einen geraden, verzweigten, gesättigten oder ungesättigten $C_1$-$C_6$ aliphatischen Rest bedeuten oder zusammen mit dem Stickstoffatom einen gesättigten oder ungesättigten Ring bilden, der noch weitere Heteroatome enthalten kann und gegebenenfalls durch eine niedrige Alkylgruppe, eine niedrige Alkoxygruppe oder ein Sauerstoffatom substituiert ist,
$R_4$ einen unsubstituierten oder einen ein- oder mehrfach substituierten monocyclischen aromatischen Rest, einen unsubstituierten oder substituierten fünf-oder sechsgliedrigen heteroaromatischen Rest bedeutet,
$R_5$ Wasserstoff, -C≡N,

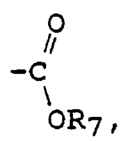

einen unsubstituierten, einen ein- oder mehrfach substituierten monocyclischen aromatischen Rest oder einen unsubstituierten oder substituierten fünf-oder sechsgliedrigen heteroaromatischen Rest bedeutet,
$R_6$ Wasserstoff, -C≡N,

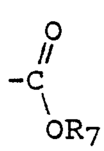

,

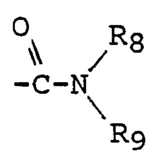

oder

$-CH_2-O-R_{10}$
$R_7$ Wasserstoff, einen $C_1$-$C_{12}$-Alkylrest oder einen N-Dialkylamino-$C_1$-$C_6$-alkyl-Rest bedeutet.
$R_8$ und $R_9$ gleich oder verschieden sein können und Wasserstoff einen geradkettigen, verzweigten, gesättigten oder ungesättigten aliphatischen $C_1$-$C_{12}$-Rest bedeuten, oder zusammen mit dem Stickstoff einen gesättigten oder ungesättigten Ring bilden mit 2 - 6 C-Atomen.
$R_{10}$ Wasserstoff, einen geradkettigen oder verzweigten $C_1$-$C_6$-Alkyl- oder $C_2$-$C_6$-Alkenylrest, einen Aralkylrest oder einen Acylrest bedeutet,
X einen Valenzstrich oder die Methylengruppe bedeutet,
Y einen Valenzstrich oder einen geradkettigen, verzweigten, gesättigten oder ungesättigten Kohlenwasserstoffrest von 1-6 Kohlenstoffatomen bedeutet, und
Z einen Valenzstrich, ein Sauerstoffatom oder die Carbonylgruppe bedeutet.
sowie deren pharmakoloigsch unbedenkliche Salze und deren optische Isomere, dadurch gekennzeichnet, daß man in ublicher Weise

a) eine Verbindung der allgemeinen Formel II,

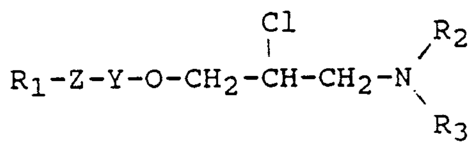
(II)

in der Y, Z, $R_1$, $R_2$ und $R_3$ die oben genannten Bedeutungen besitzen, mit einer Verbindung der allgemeinen Formel III,

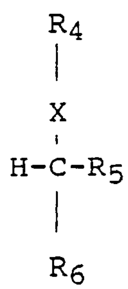
(III)

in der X, $R_4$, $R_5$ und $R_6$ die oben genannten Bedeutungen besitzen, umsetzt
oder

b) eine Verbindung der allgemeinen Formel IV,

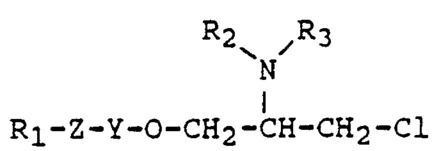

in der Y, Z, $R_1$, $R_2$ und $R_3$ die oben genannten Bedeutungen besitzen, mit einer Verbindung der allgemeinen Formel III umsetzt,
und anschließend gewunschtenfalls die erhaltenen Verbindungen in andere Verbindungen der Formel I sowie in ihre pharmakolgoisch verträglichen Salze überführt oder in optische Isomere auftrennt.

4. Arzneimittel, enthaltend eine Verbindung gemäß Anspruch 1 oder 2 neben üblichen Träger- und Hilfsstoffen.

5. Verwendung von Verbindungen gemäß Anspruch 1 oder 2 zur Behandlung von Herz- und Kreislauferkrankungen.